# EUROPÄISCHE PATENTSCHRIFT

(11) **EP 0 625 163 B1**
(45) Veröffentlichungstag und Bekanntmachung des Hinweises auf die Patenterteilung: **07.10.1998**
(21) Anmeldenummer: 93905233.8
(22) Anmeldetag: 04.02.1993
(51) Int. Cl.: C07K 14/635, A61K 38/29

(54) **PARATHORMONFRAGMENTE, DEREN HERSTELLUNG UND DIESE ENTHALTENDE ARZNEIMITTEL**
PARATHYROID HORMONE FRAGMENTS, THEIR PREPARATION AND MEDICAMENTS CONTAINING THE SAME
FRAGMENTS DE PARATHORMONE, LEUR FABRICATION ET MEDICAMENTS LES CONTENANT

(30) Priorität: 04.02.1992 DE 4203040
(43) Veröffentlichungstag der Anmeldung: 23.11.1994
(73) Patentinhaber: BOEHRINGER MANNHEIM GMBH, 68305 Mannheim (DE)
(72) Erfinder: DUVOS, Christian, D-2160 Stade (DE); MAYER, Hubert, D-3340 Wolfenbüttel (DE); MUELLER-BECKMANN, Bernd, D-6718 Grünstadt (DE); STREIN, Klaus, D-6944 Hemsbach (DE); WINGENDER, Edgar, D-3340 Wolfenbüttel (DE)
(74) Vertreter: Köster, Reinhold, Dr.
(86) Internationale Anmeldenummer: EP9300259
(87) Internationale Veröffentlichungsnummer: WO9315109

(56) Entgegenhaltungen:
- EP-A- 0 301 485
- WO-A-91/06564
- FR-A- 2 666 987
- US-A- 4 086 196

## Beschreibung

Die Erfindung betrifft neue Parathyroidhormon (PTH)-Fragmente, sowie ihre pharmakologisch verträglichen Derivate, die Herstellung dieser Fragmente und ihrer Derivate auf chemischem Weg und diese enthaltende Arzneimittel.

Das Parathyroidhormon (PTH), ein Hormon der Nebenschilddrüsen, ist ein wichtiger Regulator unter anderem zur Aufrechterhaltung des Calciumspiegels im Körper. PTH kann die Knochenbildung oder Knochenresorption stimulieren. Es wirkt dabei als regulatorisches Hormon auf eine Reihe von Enzymen, unter anderem die Ornithindecarboxylase und Adenylatcyclase (cAMP-Synthese). PTH mobilisiert bei Calciummangel Calcium aus den Knochen, verringert die Calciumausscheidung der Nieren und verbessert gleichzeitig die Resorption von Calcium aus dem Darm durch eine erhöhte Synthese von 1,25-(OH)₂D₃. Durch die Wirkung auf diese Zielorgane wird eine Normalisierung des Calciumspiegels erreicht. Umgekehrt wird bei erhöhtem Calciumspiegel der Einbau von Calcium in die Knochen stimuliert. Ferner zeigt PTH einen mitogenen Effekt, insbesondere eine Stimulation von Osteoblasten und Chondrocyten.

Aus der DE-OS 37 25 319 ist ferner bekannt, daß die oben genannten Effekte durch einen speziellen Bereich des PTH bewirkt wird und daB es möglich ist, durch Gabe von PTH-Fragmenten, welche diesem Bereich entsprechen, die gleiche Wirkung zu erzielen (siehe auch Sömjen et al., Biochem. J. (1990) 272, 781-785; Shurtz-Swirski et al., Acta Endocrinol. (1990) 122, 217-226 und Potts et al, Advances in Protein Chemistry (1982) 35, 323-396). Dieser Literatur ist auch zu entnehmen, wie solche PTH-Fragmente hergestellt und variiert werden können, wobei sowohl eine Spaltung des natürlich vorkommenden PTH als auch chemisch synthetische oder gentechnologische Verfahren möglich sind. Ein chemisch synthetisches Verfahren zur Herstellung des hPTH (1-37) ist beschrieben in der internationalen Anmeldung WO 91/06564. Diese bekannte Festphasen- und Flüssigphasensynthese hat jedoch den Nachteil, daß die Ausbeuten bei der Herstellung relativ gering sind.

PTH-Fragmente sind insbesondere für die Behandlung von Osteoporose interessant. Allerdings hat sich herausgestellt, daß die Fragmente unterschiedlich wirksam sind und daß in einigen Fällen, z.B. bei der Anwendung des hPTH(1-34) die Wirkung bei einigen Patienten positiv ist, bei anderen aber eine Verschlechterung der Calciumretention eintrat und die Gesamtknochenmasse während der Behandlung zurückging.

Es ist deshalb die Aufgabe der Erfindung, neue PTH-Fragmente zur Verfügung zu stellen, die eine verbesserte therapeutische Wirkung, insbesondere bei der Regulierung des Calciumspiegel im Körper und beim Einbau von Calcium in die Knochen haben. Außerdem sollten sich diese Fragmente mit einem relativ geringen Aufwand in guter Ausbeute herstellen lassen.

Diese Aufgabe wird mit PTH-Fragmenten gelöst ausgewäht aus der Gruppe der hPTH-Modifikationen PTH(1-35), PTH(1-36), PTH(2-35), PTH(2-36), PTH(3-35) und PTH(3-36). Insbesondere kommen im Sinne der Erfindung die PTH-Fragmente (1-35) und (1-36) in Frage. Im Sequenzprotokoll sind die Aminosäuresequenzen der folgenden Fragmente enthalten:
SEQ ID No. 1 : Kernfragment hPTH(3-35)
SEQ ID No. 2 : hPTH(3-36)
SEQ ID No. 3: hPTH(1-35)
SEQ ID No. 4: hPTH(1-36)

Gegenstand der Erfindung ist ferner ein Verfahren zur Herstellung dieser Fragmente, indem man die genannten Fragmente oder Teil fragmente davon mittels Flüssigphasen-Synthese aus geschützten Aminosäuren herstellt, die in der Reihenfolge aneinander gekoppelt werden, so daß die gewünschte Aminosäurensequenz des jeweiligen Fragmentes entsteht. Gegenstand der Erfindung sind auch Arzneimittel, die ein erfindungsgemäßes PTH-Fragment als aktiven Wirkstoff neben üblichen Hilfs- und Zusatzstoffen enthalten.

Die Aminosäuresequenzen von hPTH, bPTH und pPTH (h=human, b=bovine, p=porcine) sind bekannt aus Potts et al., Adv. in Protein Chem. (1982), 323-396; z.B. hPTH(1-34) mit der Aminosäurensequenz Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe.

Die erfindungsgemäßen PTH-Fragmente mit dem Aminosäurebereich von PTH(3-35), die ausgehend von der Aminosäureposition +3 am N-Terminus um eine oder zwei Aminosäuren verlängert sein können, sind insbesondere solche Fragmente, die in Position +2 die Aminosäure D- oder L-Val besitzen. Fragmente, die um zwei Aminosäuren am N-Terminus verlängert sind, besitzen in Position +2 die Aminosäure D-oder L-Val und in Position +1 die Aminosäure D- oder L-Ser.

Fragmente, die am C-Terminus ausgehend von der Aminosäureposition +35 (Val) um eine Aminosäure verlängert sind, stellen insbesondere solche Fragmente dar, die in Position +36 die Aminosäure D- oder L-Ala aufweisen.

Bevorzugt kommen die Fragmente PTH(1-35) mit der Aminosäuresequenz Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-Val und PTH(1-36) mit der Aminosäuresequenz Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-Val-Ala in Frage.

Die Carboxylgruppe der Aminosäure am C-terminalen Ende kann in freier Form oder in Form eines physiologisch verträglichen Alkali- oder Erdalkalisalzes, wie z.B. des Natrium-, Kalium- oder Calciumsalzes vorliegen. Die Carboxylgruppe kann auch amidiert sein mit primären oder sekundären Aminen, wie z.B. Ammoniak, C₁-C₆-Alkylamin oder Di-C₁-C₆-Alkylaminen, insbesondere Methylamin oder Dimethylamin, d.h. -CONR¹R², mit R¹ = Wasserstoff, C₁-C₆-Alkyl und R²=Wasserstoff, C₁-C₆-Alkyl, wobei die Alkylgruppen beispielsweise Methyl, Ethyl, n-Propyl, i-Propyl, Butyl, etc. bedeuten können).

Bevorzugt kommen in diesem Sinne die Fragmente PTH(2-35), PTH(2-35)-Z, PTH(1-35), PTH(1-35)-Z, PTH(2-36), PTH(2-36)-Z, PTH(1-36) oder PTH(1-36)-Z in Frage, insbesondere die entsprechenden hPTH-Fragmente, wobei Z die oben angegebene amidierte Carboxylgruppe -CONR¹R² bedeutet. Insbesondere bedeutet R¹ ein Wasserstoffatom und R² eine Methyl-, Ethyl- oder Isopropylgruppe.

Die erfindungsgemäßen PTH-Fragmente besitzen vorteilhafte therapeutische Eigenschaften. Insbesondere läßt sich mit ihnen nicht nur der Calciumspiegel im Körper regulieren, sondern auch der Einbau von Calcium in die Knochen gezielt fördern, weshalb sie den Verlauf der Osteoporose günstig beeinflussen bzw. diese zum Stillstand bringen können. Diese Wirkung ist überraschend, weil diese PTH-Fragmente, anders als die bekannten Fragmente hPTH(1-37) oder hPTH(1-34), nicht zu den biologisch aktiven hPTH-Fragmenten gehören, welche durch primäre Spaltung des hPTH(1-84) im menschlichen Körper entstehen. Als weiterer Vorteil der erfindungsgemäßen PTH-Fragmente kommt hinzu, daß sie sich in besserer Ausbeute als die Fragmente mit längerer Aminosäurensequenz herstellen lassen.

Gegenüber den bekannten Fragmenten hPTH(1-n) mit n=34, 37 oder 38 besitzen die erfindungsgemäßen PTH-Fragmente eine überraschend höhere Wirksamkeit in verschiedenen Testsystemen, so daß sie für eine therapeutische Anwendung besser geeignet sind als die bisher bekannten Fragmente. Die Vorteile der neuen Fragmente sind insbesondere die folgenden: stärkere Mitogenität und DNA-Syntheseleistung; geringere katabole, calciummobilisierende Wirkung; geringere cAMP-Aktivierung; Steigerung der Calciumretention und verstärkter Calciumeinbau in die Knochen. Diese Effekte bewirken insgesamt eine deutlich überlegene anabole Wirkung der neuen PTH-Fragmente im Vergleich zu bisher bekannten PTH-Fragmenten.

Die Festphasen- und Flüssigphasensynthese ist ein übliches, auch schon für die Synthese von hPTH-Fragmenten angewandtes Verfahren (siehe WO 91/06564). Nachteilig bei diesem Verfahren ist jedoch, daß auch viele Verunreinigungen erzeugt werden, welche nicht nur die Reindarstellung erschweren, sondern auch die Ausbeute erniedrigen.

Um das Verfahren für die Herstellung eines bestimmten Produktes im Hinblick auf die Reinheit des Rohproduktes und die Ausbeute zu optimieren, ist es erforderlich, die Prozeßparameter und die verwendeten Materialien, beispielsweise die Materialien für das Blockieren der Gruppen, welche nicht reagieren sollen, oder die Reagenzien, welche blockierende Materialien abspalten, an das herzustellende Produkt, an die herzustellenden Zwischenprodukte bzw. Ausgangsmaterialien anzupassen. Diese Anpassung ist angesichts der Interdependenz der vielen Verfahrensparameter nicht einfach. Bei der Herstellung der erfindungsgemäßen PTH-Modifikationen wirkt es sich vorteilhaft aus, wenn das für die Abtrennung des synthetisierten Peptids von der Festphase und der Seitengruppenblockierungen verwendete "Reagens K" als Bestandteil Dithiothreitol (DTT) statt Ethandithiol enthält.

Es hat sich gezeigt, daß auch das aus dem Artikel "Simultaneous Multiple Peptide Synthesis under Continuous Flow Conditions on Cellulose Paper Discs as Segmental Solid Supports" von R. Frank i. a., veröffentlicht in Tedrahedron Vol. 44, No. 19, pp 603-604 (1988), bekannte Filterverfahren prinzipiell für die Herstellung von beispielsweise hPTH(1-35) und hPTH(1-36) geeignet ist, jedoch PTH-Fragmente mit Aminosäuresequenzen, die über die Zahl 36 hinausgehen mit dieser Methode - offenbar aufgrund der Bildung von großen Mengen von Verunreinigungen - nur mit deutlich reduzierter Ausbeute in reiner Form erzeugt werden können.

Arzneimittel, welche die erfindungsgemäßen Fragmente einzeln oder zusammen als aktiven Wirkstoff neben üblichen Hilfs- und Zusatzstoffen enthalten, wurden vorzugsweise parenteral verabreicht. Das Arzneimittel hat eine calciumregulierende Wirkung und fördert dabei in vorteilhafter Weise den Einbau von Calcium in die Knochen. Vorteilhaft für die Anwendung des Arzneimittels ist es, wenn es den Wirkstoff in Mengen von 300 Mikrogramm bis 30 Milligramm pro Dosiseinheit enthält. Eine gute Wirkung wird insbesondere dann erzielt, wenn die Dosiseinheit an erfindungsgemäßem Fragment 100 bis 1.000 Einheiten bzw. einer wirksamen Konzentration von 10⁻⁸ - 10⁻⁷ M/l Körperflüssigkeit entspricht.

Die erfindungsgemäßen PTH-Fragmente werden vorzugsweise als sterile Lyophilisate gelagert und vor der Applikation mit einer geeigneten isotonischen Lösung vermischt. In dieser Form können die Fragmente dann injiziert, infundiert oder gegebenenfalls auch durch die Schleimhäute absorbiert werden. Als Lösungsmittel können die üblichen, für die Injektion oder Infusion geeigneten isotonischen, wässrigen Systeme verwendet werden. Physiologische Kochsalzlösung oder gegebenenfalls durch Puffer isotonisch gestellte Lösungen werden in diesem Fall bevorzugt.

Die zu verabreichende Tagesdosis hängt von der Indikation ab. Bei der Therapie der Osteoporose durch i.v./i.m. Injektion liegt sie im Bereich von 100 bis 1.200 Einheiten (µg)/Tag, bei täglicher subcutaner Injektion vorzugsweise bei 300 - 2.400 Einheiten (µg)/Tag. Die Bestimmung der biologischen Aktivität basiert auf Messungen gegen internationale Referenzpräparationen für hPTH-Fragmente in einem gebräuchlichen biologischen Testverfahren für hPTH-Fragmente.

Die erfindungsgemäßen PTH-Fragmente können nach dem Filtersynthese-Verfahren oder dem konventionellen Protokoll über einen kommerziellem Synthesizer hergestellt werden.

### Beispiel 1

### Chemische Synthese von hPTH(1-35) und hPTH(1-36)

Bei der Herstellung nach dem erfindungsgemäßen Verfahren (chemische Synthese) wurde in vorteilhafter Weise ein Milligen 9050 Peptid-Synthesizer verwendet. Es wurden Pentafluorophenylester der Aminosäuren eingesetzt, in Kombination mit Hydroxy-benzotriazol (HOBt) zur Verhinderung von Razemisierungen während der Kopplungsreaktion. N-alpha-Aminofuktionen waren durch die Fluorenylmethoxycarbonylgruppe (Fmoc) geschützt. Aminosäure-Seitenketten waren durch t-Boc (Histidin und Lysin), t-But (Asparaginsäure, Glutaminsäure und Serin) und Pmc (2,2,5,7,8-Pentamethylchroman-6-sulfonyl) (Arginin) geschützt. Bevorzugt wurden als Harze verwendet: Fmoc-Val-NovaSyn-PA 500 (Novabiochem), Substitutionsgrad 0,37 mMol/g für hPTH (1-35) und Fmoc-Ala-NovaSyn-PA 500 (Novabiochem), Substitutionsgrad 0,35 mMol/g für hPTH (1-36). Die Abspaltung der Peptide vom Harz und die Schutzgruppenabspaltung wurde mit einer Mischung aus Trifluoressigsäure (TFA), Phenol, Wasser, Thioanisol und Dithiothreitol (DTT) vorgenommen. Bevorzugt ließ man dabei für jeweils etwa zwei Stunden eine Mischung aus 82,5 % TFA, 5 % Phenol, 5 % Wasser, 5 % Thioanisol und 2,5 % DTT und modifiziertes Reagens K (s. King et al., Int. J. Peptide Protein Res. 36, 255-266, 1990) einwirken. Die Fmoc-Gruppe wurde mittels einer Lösung von Piperidin in Dimethylformamid (DMF) abgespalten. Bevorzugt war die Lösung 20 %ig. Die Rohpeptide wurden mit t-Butylmethylether gefällt, in Wasser/Aceto-nitril - bevorzugt - 9:1 gelöst, - bevorzugt - über C-18 Kartuschen (J. T. Baker Chemical Co.) vorgereinigt und lyophilisiert.

Die Ausbeute betrug bei Herstellunq unter den bevorzugten Bedingungen 71 % für hPTH (1-35) und 67 % für hPTH (1-36). Die Rohpeptide wurden durch präparative HPLC - bevorzugt -auf reversed phase-C4 (RP-4)-Material mit einem Wasser-Acetonitril Gradienten gereinigt. Die Ausbeute beträgt 10-15 %, bezogen auf die Rohpeptide.

### Beispiel 2

### Herstellung von hPTH(1-35) und hPTH(1-36) nach der Filtermethode

Die Synthese bei Anwendung der Filtermethode erfolgte nach der Fmoc/t-Butyl (tBu)-Methode unter Verwendung der 1-Hydroxybenzotriazol (HOBt)/Diisopropylcarbodiimid (DICD)-Kopplung. Als spezielle Seitenkettenschutzgruppen wurden Pmc für Arg, Trimethoxybenzyl (Tmob) für Asn und Gln und Butoxycarbonyl (Boc) für His eingesetzt.

Bei der Herstellung der erfindungsgemäßen PTH-Fragmente nach der Filtermethode wurden je Sequenz zwei Filter mit einer Beladung von je 5 µMol C-terminalem Aminosäurederivat beladen. Jede Sequenz wurde also im Maßstab 10 µMol hergestellt.

Vor Beginn der Synthese wurden zunächst die Cellulosefilter für die Aufnahme der Aminosäuren vorbereitet. Dazu wurde das Filterpapier durch eine schonende Säurebehandlung z. B. mit einer 10 %igen Lösung von TFA in Dichlormethan zum Quellen gebracht und anschließend mit einer "Ankopplungsverbindung" umgesetzt. Bevorzugt wurde eine Kopplung vom Benzylester-Typ, um die erste Aminosäure an dem Träger zu verankern. Besonders günstig als "Ankopplungsverbindung" - auch im Hinblick auf die spätere Abtrennung - war ein p-Alkoxybenzylderivat mit der Formel I,

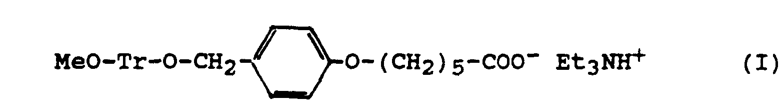

welches eine 4-Methoxytritylether-Gruppe als schützende Gruppe aufweist, die leicht mit Dichloressigsäure in Dichlormethan abgespalten werden kann. Herstellen läßt sich die Verbindung durch selektive Tritylierung von 4-Hydroxyphenol mit 4-Methoxytritylchlorid. Die Methoxytritylgruppe wird als Schutzgruppe bei der Veresterung der Cellulose-Hydroxylgruppen mit dem Alkoxybenzylderivat benötigt.

Die Anbringung der ersten Fmoc-Aminosäure wurde wie bei anderen mit Benzylalkohol substituierten Trägern durchgeführt, indem ein dreifacher Überschuß über die Filterbeladung an voraktivierten Aminosäurederivaten unter Verwendung von Dimethylaminopyridin (DMAP) als Katalysator mit der umgesetzten Unterlage zur Reaktion gebracht wurde. Die Voraktivierung wurde erreicht, indem die Fmoc-geschützten Aminosäurederivate mit 1-Hydroxybenzotriazol (HOBt) und Diisopropylcarbodiimid (DICD) zum HOBT-Ester umgesetzt wurden. Bevorzugt wurden die Aminosäurederivate in DMF mit 1,5 Äquivalent HOBt und 1,2 Äquivalent DICD 45 Minuten lang umgesetzt. Die voraktivierten Aminosäurederivate wurden in situ auf die Filterpapiere gegeben, wo die Aminosäure mit dem Säureende angekoppelt wurde. Zur Vorbereitung der Ankopplung der zweiten und der weiteren Aminosäuren wurde die Fmoc-Gruppe der letzten angekoppelten Aminosäure mit einer - bevorzugt - 20 %igen Lösung von Piperidin in DMF abgespalten. Anschließend erfolgte dann die Ankopplung der nächsten Fmoc-geschützten Aminosäure, die Amidbildung wurde durch den Bromphenolblau-Indikator (s. V. Krchnak i. a. Collect. Czech. Chem. Commun., 53, 2542 (1988)) überprüft und war nach 0,5 bis 2 Stunden vollständig.

Nach dem Abschluß der Peptid-Synthese erfolgte die TFA-geförderte Abtrennung des Peptids von dem Alkoxybenzylalkohol-Derivat. Dabei wurden auch die t-But-Gruppen für den Seitengruppenschutz abgetrennt. Bevorzugt wurde hierfür ein TFA/Anisol/Dichlormethan-Gemisch verwendet, dessen optimale Zusammensetzung 55 Volum-% TFA, 5 Volum-% Anisol und 40 Volum-% Dichlormethan war.

Die abgespaltenen Rohpeptide wurden mit Ether extrahiert und anschließend lyophilisiert. Die Ausbeute betrug 43 mg für hPTH(1-35) und 34 mg für hPTH(1-36). Das so hergestellte Rohpeptid wurde durch präparative RP-Chromatographie - bevorzugt - an C4-Kieselgel gereinigt.

### Beispiel 3

### Bestimmung der Wirkung verschiedener PTH-Fragmente auf die Ca-Retention bei Ratten

Die Messung der Ca-Bilanz bei wachsenden Ratten ist ein geeignetes Modell, die Wirksamkeit einer systemisch verabreichten Substanz auf den Knochenstoffwechsel in vivo zu testen. Bei der Ca-Bilanz werden die Ca-Ströme in und aus dem Körper gemessen und daraus die Ca-Retention (Bilanz) berechnet. Dabei wird berücksichtigt, daß der Knochen ca. 99 % des Körpercalciums speichert, während der Plasmacalciumspiegel konstant bleibt. Die aus der Nahrung im Körper zurückbehaltende Calciummenge kann daher als proportional zum Zuwachs an Knochenmasse angesehen werden.

Als Versuchstiere für die Untersuchungen dienten männliche CD-Ratten, die zu Versuchsbeginn 10 Wochen alt waren und 250 - 300 g wogen. Die Versuchstiere wurden in handelsüblichen Stoffwechselkäfigen einzeln gehalten. In den Stoffwechselkäfigen erfolgte eine automatische Trennung von Fäzes und Urin. Beide Fraktionen wurden in Auffangbehältern quantitativ gesammelt und konnten getrennt voneinander analysiert werden.

Als Futter diente eine Standarddiät für Ratten von mehlförmiger Konsistenz, das mit Aqua dest. im Verhältnis 1:1 (W/W) angeteigt wurde. Die täglich verabreichte Futtermenge wurde im Sinne eines sogenannten "group feedings" dem Futterbedarf der Tiere so angepaßt, daß sie von allen Tieren vollständig gefressen wurden. Die tägliche Futterration betrug 20 g (Naßfutter). Aqua dest. wurde den Tieren ad libitum angeboten.

Alle Testsubstanzen wurden täglich in äquimolaren Dosen (entsprechend 40 g hPTH (1-34) x kg⁻¹ x Tag⁻¹) subkutan über 10 Tage injiziert. Davon dienten die ersten 7 Tage zur Adaptation der Tiere, während die Messung der Ca-Bilanz während der letzten 3 Tage stattfand.

Der Ca-Intake wurde über die Menge aufgenommenen Futters und dem experimentell bestimmten Ca-Gehalt des Futters berechnet. Dazu wurde von der Futtercharge eines jeden Tages ein Aliquot bis zur Gewichtskonstanz getrocknet und aus dem Vergleich von Naß- und Trockengewicht der Wassergehalt bestimmt. Anschließend wurden 2 x 500 mg des getrockneten Futters mit einem Labormikrowellengerät in konz. Säuren wie folgt aufgeschlossen: Zu 500 mg getrocknetem Futter wurden nacheinander 3 ml HNO₃ (konz.), 1 ml HCl (konz.) und 1 ml H₂O₂ (30 %) pipettiert. Nach dem Abklingen der spontanen Oxidationsreaktion (Aufschäumen) erfolgt der Aufschluß druckgesteuert in abgeschlossenen Aufschlußbehältern aus Teflon PFA. Dabei wurden nacheinander folgende Druck-/Zeitstufen durchlaufen: 40 psi (276 kPa), 5 min; 80 psi (552 kPa), 5 min; 150 psi (1.035 kPa) ; 10 min. Dem abgekühlten Aufschluß wurden nochmals 2 ml H₂O₂ (30 %) zugegeben, um die Vollständigkeit der Aufschlußreaktion zu überprüfen (kein Aufschäumen mehr). Der Aufschluß wurde mit einer 1 %igen Lanthannitratlösung (V/V in 0,3 %iger HCl) ad 100 ml aufgefüllt. Zur Messung des Ca-Gehaltes am Flammen-Atomabsorptionsspektrometer (AAS) wurden die Proben nochmals 1:100 mit 1 %iger Lanthannitratlösung verdünnt werden. Aus der aufgenommenen Futtermenge und dem gemessenen Wasser- und Ca-Gehalt wurde dann der Ca-Intake berechnet.

Der Ca-Verlust über die Fäzes wurde analog zum Ca-Intake ermittelt: Bestimmung des Wassergehaltes, saurer Aufschluß im Mikrowellengerät unter gleichen Bedingungen und anschließende Messung des Ca-Gehaltes am AAS.

Im Sammelbehälter für den Urin wurde 2 ml 10 %ige HCl vorgelegt, um einer Ausfällung von Urat vorzubeugen. Nach Versuchsende wurde das Urinvolumen durch Wägung bestimmt und anschließend ein Aliquot davon mit 1 %iger Lanthannitrat Lösung (V/V in 0,3 %iger HCl) im Verhältnis 1:100 verdünnt. Die Bestimmung des Ca-Gehaltes der Proben erfolgte am AAS.

### Ergebnis:

Die Ergebnisse sind in der folgenden Tabelle wiedergegeben:

**Tab. 1:**

| Ca-Retention (mg pro Tag) | | | |
|---|---|---|---|
| Substanz | Mittelwert | +/- SD | % Zunahme |
| Kontrolle | 49,5 | 5,32 | 0 |
| hPTH (1-34) | 91,8 | 10,25 | 85 |
| hPTH (1-35) | 108,6 | 9,21 | 119 |
| hPTH (1-36) | 110,5 | 11,44 | 123 |
| hPTH (1-37) | 96,0 | 7,69 | 94 |
| hPTH (1-38) | 91,8 | 9,68 | 85 |

### Bewertung:

Überraschenderweise wurde gefunden, daß die Parathormonfragmente mit einer Kettenlänge von 35 oder 36 Aminosäuren - entsprechend den Peptidsequenzen PTH (1-35) und PTH (1-36) zu einer höheren Ca-Retention und deutlicher ausgeprägten Einbaurate von Calcium in den Knochen führen, als die aus dem Stand der Technik bekannten Peptide hPTH (1-n) mit n=34,37,38. Eine gesteigerte Ca-Retention wurde auch gefunden, wenn die Peptide N-terminal um eine Aminosäure verkürzt waren.

### Beispiel 4

### Mitogenität an Sterna-Organkulturen

In Organkulturen aus Sterna wurde die Mitogenität der neuen Fragmente geprüft und festgestellt, daß diese Aktivität stärker ausgeprägt ist als bei den bekannten Fragmenten hPTH(1-34) und hPTH(1-38) (vgl. Tab. 2).

**Tab. 2:**

| Mitogenität an Sterna-Organkulturen | | |
|---|---|---|
| | n | E/C +/- SEM |
| Kontrolle | 4 | 1.00 +/- 0.13 |
| hPTH(1-34) | 4 | 2.94 +/- 0.38 |
| hPTH(1-35) | 4 | 3.13 +/- 0.57 |
| hPTH(1-36) | 4 | 3.37 +/- 0.58 |
| hPTH(1-38) | 4 | 2.96 +/- 0.30 |

### Beispiel 4

### DNA-Syntheseleistung an ROS 17/2.8-Zellen

An ROS 17/2.8-Zellen wurde die DNA-Syntheseleistung gemessen. Überraschend wurde die Syntheseleistung durch das Fragment PTH(1-35) stärker gesteigert als durch andere Fragmente (vgl. Tab. 3).

**Tab. 3:**

| DNA-Synthese hPTH(1-X), 100 nM, ROS/2.8 Zellen | | | |
|---|---|---|---|
| X | n | [³H]Thymidin-Einbau (cpm) | E/C |
| (Basalwert) | 4 | 663 ± 49 | 1.0 ± 0.07 |
| 34 | 5 | 2.148 ± 289 | 3.2 ± 0.44 |
| 35 | 6 | 2.686 ± 282 | 4.1 ± 0.43 |
| 36 | 6 | 2.136 ± 246 | 3.2 ± 0.37 |
| 37 | 6 | 2.021 ± 251 | 3.1 ± 0.38 |
| Die Induktion durch hPTH(1-35), hPTH(1-36) und hPTH(1-37) mit p<0.001 gegenüber der Basalrate erhöht, 1-34 mit p<0.01. | | | |

### Beispiel 5

### cAMP-Stimulation

In Organkulturen aus Sterna sowie an Membranfraktionen aus Schwienenieren wurde die Induktion der cAMP-Bildung durch verschiedene PTH-Peptide gemessen. überraschend nimmt diese Aktivität für die Fragmente PTH(1-35) und PTH(1-36) ab verglichen mit PTH(1-34) (vgl. Tab. 4 und Tab. 5). Dies ist von Bedeutung, da die katabolen, d.h. calciummobilisierenden Eigenschaften des PTH bekanntermaßen mit einer Steigerung des cAMP einhergehen (Schlüter et al. (1989), J.Biol.Chem. 264,19:11087-11092; Löwik et al., (1988), Calcif Tissue Int. 43:7-18). Diese Befunde sind plausibel und stützen deshalb die o.g. Befunde, die eine Zunahme der anabolen Eigenschaften des Peptids bei einer Kettelänge zwischen 35 und 36 Aminosäuren zeigen.

**Tab. 4:**

| cAMP-Stimulation an Sterna-Organkulturen 100 nM Induktor | | |
|---|---|---|
| | n | pMol cAMP |
| Kontrolle | 4 | 4.7 |
| 1-34 | 4 | 25.8 |
| 1-35 | 4 | 18.2 |
| 1-36 | 4 | 17.6 |

**Tab. 5:**

| cAMP-Stimulation an Schweinenierenmembran | |
|---|---|
| | pMol cAMP/mg Protein ± SEM |
| Basalsynthese | 20.6 ± 1.2 |
| hPTH(1-34) | 86.6 ± 1.0 |
| hPTH(1-35) | 82.4 ± 0.4 |
| hPTH(1-36) | 44.6 ± 1.8 |
| mit jeweils n=3 und p<0.001. | |

### Beispiel 6

### DNA-Syntheseleistung an PTH-(3-X)-Fragmenten

Eine gesteigerte DNA-Syntheseleistung wurde an UMR 106 Zellen auch mit Fragmenten beobachtet, deren N-Terminus um zwei Aminosäuren verkürzt war. Ein Wirkungsmaximum lag wiederum vor, wenn die Kettenlänge zwischen 34 und 36 Aminosäuren lag (vgl. Tab. 6). Dieser Befund ist überraschend, da bekannt ist, daß eine Deletion der Aminosäuren +1 oder +1 und +2 zum Verlust der cAMP-Induktion führt, verbunden mit dem Verlust der calciummobilisiernden Eigenschaften des PTH. Eine anabole (mitogene) Aktivität von N-terminal verkürzten PTH-Fragmente ist bisher nicht beschrieben worden.

**Tab. 6a:**

| DNA-Synthese PTH(3-X), 100 nM, UMR 106 Zellen | | | |
|---|---|---|---|
| X | n | [³H]Thymidin-Einbau (cpm) | E/C |
| Basalwert | 5 | 925 ± 48 | 1.0 ± 0.04 |
| 34 | 5 | 1.748 ± 150 | 1.9 ± 0.12 |
| 35 | 4 | 2.591 ± 115 | 2.8 ± 0.1 |
| 36 | 5 | 2.230 ± 172 | 2.4 ± 0.14 |
| 37 | 5 | 2.161 ± 74 | 2.3 ± 0.06 |
| 38 | 5 | 1.905 ± 159 | 2.1 ± 0.13 |
| Alle Induktionen mit p<0.001 gegenüber der Basalrate erhöht. | | | |

**Tab. 6b:**

| DNA-Synthese PTH(3-X), 300 nM, UMR 106 Zellen | | | |
|---|---|---|---|
| X | n | [³H]Thymidin-Einbau | E/C |
| Basalwert | 4 | 972 ± 32 | 1.0 ± 0.03 |
| 34 | 5 | 3.516 ± 297 | 3.6 ± 0.31 |
| 35 | 5 | 4.158 ± 213 | 4.3 ± 0.22 |
| 36 | 5 | 4.950 ± 401 | 5.1 ± 0.41 |
| 37 | 5 | 3.962 ± 268 | 4.1 ± 0.28 |
| 38 | 5 | 3.701 ± 414 | 3.8 ± 0.43 |
| Alle Induktionen mit p<0.001 gegenüber der Basalrate erhöht. | | | |

### Beispiel 7

### Synthese von PTH (1-35), PTH (1-35)-Z mit Z = NH₂, NH(C₂C₅)

Die Synthese der Peptide wurde nach der Festphasenmethode durchgeführt und zwar mit folgenden Schutzgruppen:

### Schutzgruppen:

| | |
|---|---|
| -Aminogruppen | Fmoc |
| His (Im) | Trityl |
| Asp ( COOH) | OtBu |
| Glu ( COOH) | OtBu |
| Lys ( -NH₂) | BOC |
| Ser (OH) | tBu |
| Arg (Gua) | Pmc |

Die Amid-Funktionen von Asn und Gln bleiben ungeschützt.

### Beladung des Harzes:

25 g p-Benzyloxybenzylalkohol-Harz ("Alkoxy-Harz", Novabiochem) wurden mit 13,5 g Fmoc-ValOH und 8,5 g DCC (41 mMol) in DMF/Dichlormethan (3:7) unter Zugabe von 0,5 g (3,9 mMol) 4-Dimethylaminopyridin beladen.

Nach Blockierung der überschüssigen freien OH-Gruppen am Harz mittels Benzoylchlorid/Pyridin und Waschen mit DMF, Isopropanol und Diisopropylether (2 Cyclen) und Trocknen erhielt man 32 g Fmoc-Val-Harz.

Nach Abspaltung der Schutzgruppe mit 20 % Piperidin in DMF ergab sich eine Beladung von 0,66 mMol/g (indirekte Bestimmung durch UV-Messung bei 300 nm in der Waschflüssigkeit über die Abspaltungsprodukte [J. Maienhofer, C.D. Chang Int. J. Pept. Prot. Res 11, 246 (1978)].

Wasch- und Synthese-Cyclen:

| | | |
|---|---|---|
| 1. | DMF | 2x5 Min. |
| 2. | Fmoc-Abspaltung 20 % Piperidin in DMF | 10 Min. |
| 3. | Fmoc-Abspaltung 20 % Piperidin in DMF | 20 Min. |
| 4. | Waschen DMF | 4x5 Min. |
| 5. | Waschen 2-Propanol | 2x5 Min. |
| 6. | Waschen DMF | 2x5 Min. |
| 7. | Kupplung (Aminosäure und HOBt 2 eq) | 5 Min. |
| 8. | Kupplung (DCC 2 eq) | 60 Min. |

Nach Schritt 5 wird die gesammelte Waschlösung jeweils zur Bestimmung der Beladung benutzt.

Der Kupplungserfolg wird qualitativ über den Ninhydrin-Test (Kaiser-Test) nach Entnahme einer kleinen Harzmenge getestet [E. Kaiser et al Anal. Biochem. 34, 595 /(1970)]. Eine evtl. Kupplungswiederholung war bei der durchgeführten Synthese nicht nötig.

### Abspaltung der Schutzgruppen und Abspaltung vom Harz

Am Ende der Synthese wurde der Ansatz in 3 Teile geteilt und diese gesondert aufgearbeitet.
a) hPTH (1-35), (Säure: PTH (1-35)-Z mit Z = COOH)
   Die Abspaltung der Schutzgruppen und die Abspaltung vom Harz erfolgte in einem Schritt mittels Trifluoressigsäure/Phenol/Wasser/Thioanisol/Ethandithiol (16,5:1:1:1:0,5).
b) hPTH (1-35)-Amid, (PTH (1-35)-Z mit Z = CONH₂)
   Dieses wurde durch Behandlung des Peptidharzes mit NH₃ in DMF (1:1) und anschließender Schutzgruppenabspaltung wie oben erhalten.
c) hPTH (1-35)-Ethylamid (PTH(1-35)-Z mit Z = CONH C₂C₅)
   Dieses wurde durch Behandlung des Peptidharzes mit 70 %igem wässrigem Ethylamin und anschließender Schutzgruppenabspaltung wie oben erhalten.

Nach Einengen im Vakuum wurde jeweils mit absolutem Ether gefällt. Die Roh-Peptide zeigten bei der HPLC-Analyse einen Gehalt an Endprodukt von 35 % (Säure und Ethylamid) bzw. 30 % (Amid) (Flächenprozent).

Analyse der Fragmente a) - c):

HPLC-System: Gradient aus A (Wasser, 0,1 % an TFA) und B (Wasser 35/Acetonitril 65, 0,1 %ig an TFA). Start mit 100 % A linear in 40 Min auf 100 % B. Säule Hypersil ODS 5 250 x 4,6 mm.

Die präparative Aufreinigung erfolgte unter analogen Bedingungen mittels präparativer HPLC (Nova-Prep, Merck).

Man erhielt 2,7 g hPTH (1-35) Säure, 2,6 g hPTH (1-35)-Ethylamid und 2,1 g hPTH (1-35)-Amid. Reinheit lt. HPLC >95%.

## Patentansprüche (Patentansprüche für folgende(n) Vertragsstaat(en): BE, DE, DK, ES, FR, GB, GR, NL, SE)

1. hPTH-Modifikationen ausgewählt aus der Gruppe bestehend aus PTH(2-35), PTH(2-36), PTH(3-35) und PTH(3-36).

2. hPTH-Modifikationen gemäß Anspruch 1, dadurch gekennzeichnet, daß die PTH-Modifikationen PTH(2-35) oder PTH(2-36) sind.

3. hPTH-Modifikationen gemäß Anspruch 1, dadurch gekennzeichnet, daß die PTH-Modifikationen PTH(3-35) oder PTH(3-36) sind.

4. hPTH-Modifikationen gemäß einem der Ansprüch 1-3, dadurch gekennzeichnet, daß die C-terminale Carboxygruppe mit einer Gruppe Z amidiert ist und Z eine Gruppe der Formel -CONR¹R² darstellt, wobei R¹ und R² gleich oder verschieden sind und Wasserstoff oder eine C₁-C₆-Alkylgruppe bedeuten.

5. hPTH-Modifikation gemäß Anspruch 4, dadurch gekennzeichnet, daß die PTH-Modifikationen PTH(2-35)-Z, PTH(2-36)-Z, PTH(3-35)-Z oder PTH(3-36)-Z sind.

6. Verfahren zur Herstellung von hPTH-Modifikationen gemäß einem der Ansprüche 1-5, dadurch gekennzeichnet, daß man die Fragmente nach der Festphasen- und Flüssigphasen-Synthese aus teilweise blockierten, in den Fragmenten enthaltenden Aminosäuren herstellt, die in den Reihenfolgen aneinander gekoppelt werden, die den Aminosäurensequenzen in den Fragmenten entsprechen.

7. Arzneimittel enthaltend hPTH-Modifikationen gemäß einem der Ansprüche 1-5 als aktiven Wirkstoff neben üblichen Hilfs- und Zusatzstoffen.

8. Arzneimittel gemäß Anspruch 7 mit calciumregulierender Wirkung.

9. Verwendung von hPTH-Modifikationen gemäß einem der Ansprüche 1-5 zur Herstellung von Arzneimitteln zur Regulierung des Calciumspiegels im Körper oder zum Einbau von Calcium in die Knochen.

## Patentansprüche (Patentansprüche für folgende(n) Vertragsstaat(en): AT, CH, IE, IT, LI, LU, PT)

1. hPTH-Modifikationen ausgewählt aus der Gruppe bestehend aus PTH(1-35), PTH(1-36), PTH(2-35), PTH(2-36), PTH(3-35) und PTH(3-36).

2. hPTH-Modifikationen gemäß Anspruch 1, dadurch gekennzeichnet, daß die PTH-Modifikation PTH(1-35) ist:

3. hPTH-Modifikationen gemäß Anspruch 1, dadurch gekennzeichnet, daß die PTH-Modifikation PTH(1-36) ist.

4. hPTH-Modifikationen gemäß Anspruch 1, dadurch gekennzeichnet, daß die PTH-Modifikationen PTH(2-35) oder PTH(2-36) sind.

5. hPTH-Modifikationen gemäß Anspruch 1, dadurch gekennzeichnet, daß die PTH-Modifikationen PTH(3-35) oder PTH(3-36) sind.

6. hPTH-Modifikationen gemäß einem der Ansprüche 1-5, dadurch gekennzeichnet, daß die C-terminale Carboxygruppe mit einer Gruppe Z amidiert ist und Z eine Gruppe der Formel -CONR¹R² darstellt, wobei R¹ und R² gleich oder verschieden sind und Wasserstoff eine C₁-C₆-Alkylgruppe bedeuten.

7. hPTH-Modifikation gemäß Anspruch 6, dadurch gekennzeichnet, daß die PTH-Modifikationen PTH(1-35)-Z oder PTH(1-36)-Z sind.

8. hPTH-Modifikation gemäß Anspruch 6, dadurch gekennzeichnet, daß die PTH-Modifikationen PTH(2-35)-Z, PTH(2-36)-Z, PTH(3-35)-Z oder PTH(3-36)-Z sind.

9. Verfahren zur Herstellung von hPTH-Modifikationen gemäß einem der Ansprüche 1-8, dadurch gekennzeichnet, daß man die Fragmente nach der Festphasen- und Flüssigphasen-Synthese aus teilweise blockierten, in den Fragmenten enthaltenden Aminosäuren herstellt, die in den Reihenfolgen aneinander gekoppelt werden, die den Aminosäurensequenzen in den Fragmenten entsprechen.

10. Arzneimittel enthaltend hPTH-Modifikationen gemäß einem der Ansprüche 1-8 als aktiven Wirkstoff neben üblichen Hilfs- und Zusatzstoffen.

11. Arzneimittel gemäß Anspruch 10 mit calciumregulierender Wirkung.

12. Verwendung von hPTH-Modifikationen gemäß einem der Ansprüche 1-8 zur Herstellung von Arzneimitteln zur Regulierung des Calciumspiegels im Körper oder zum Einbau von Calcium in die Knochen.

## Claims (Claims for the following Contracting State(s): BE, DE, DK, ES, FR, GB, GR, NL, SE)

1. hPTH modifications selected from the group consisting of PTH(2-35), PTH(2-36), PTH(3-35) and PTH(3-36).

2. hPTH modifications according to claim 1, characterised in that the PTH modifications are PTH(2-35) or PTH(2-36).

3. hPTH modifications according to claim 1, characterised in that the PTH modifications are PTH(3-35) or PTH(3-36).

4. hPTH modifications according to one of claims 1 - 3, characterised in that the C-terminal carboxyl group is amidated with a group Z and Z represents a group of the formula -CONR¹R², whereby R¹ and R² are the same or different and signify hydrogen or a C₁-C₆-alkyl group.

5. hPTH modifications according to claim 4, characterised in that the PTH modifications are PTH(2-35)-Z, PTH(2-36)-Z, PTH(3-35)-Z or PTH(3-36)-Z.

6. Process for the preparation of hPTH modifications according to one of claims 1 - 5, characterised in that one prepares the fragments according to the solid phase and liquid phase synthesis from partly blocked amino acids contained in the fragments which are coupled with one another in the sequence which corresponds to the amino acids sequences in the fragments.

7. Medicament containing hPTII modifications according to one of claims 1 - 5 as active materials, besides usual adjuvants and additive materials.

8. Medicament according to claim 7 with calcium-regulating action.

9. Use of hPTH modifications according to one of claims 1 - 5 for the preparation of medicaments for the regulation of the calcium level in the body or for the incorporation of calcium into the bones.

## Claims (Claims for the following Contracting State(s): AT, CH, IE, IT, LI, LU, PT)

1. hPTH modifications selected from the group consisting of PTFI(1-35), PTH(1-36), PTH(2-35), PTH(2-36), PTH(3-35) and PTH(3-36).

2. hPTH modifications according to claim 1, characterised in that the PTH modification is PTH(1-35).

3. hPTH modifications according to claim 1, characterised in that the PTH modification is PTH(1-36).

4. hPTH modifications according to claim 1, characterised in that the PTH modifications are PTH(2-35) or PTH(2-36).

5. hPTH modifications according to claim 1, characterised in that the PTH modifications are PTH(3-35) or PTH(3-36).

6. hPTH modifications according to one of claims 1 - 5, characterised in that the C-terminal carboxyl group is amidated with a group Z and Z represents a group of the formula -CONR¹R², whereby R¹ and R² are the same or different and signify hydrogen or a C₁-C₆-alkyl group.

7. hPTH modification according to claim 6, characterised in that the PTH modifications are PTH(1-35)-Z or PTH(1-36)-Z.

8. hPTH modifications according to claim 6, characterised in that the PTH modifications are PTH(2-35)-Z, PTH(2-36)-Z, PTH(3-35)-Z or PTH(3-36)-Z.

9. Process for the preparation of hPTH modifications according to one of claims 1 - 8, characterised in that one produces the fragments according to the solid phase and liquid phase synthesis from partly blocked amino acids contained in the fragments which are coupled with one another in the sequence which corresponds to the amino acid sequences in the fragments.

10. Medicaments containing hPTH modifications according to one of claims 1 - 8 as active material, besides usual adjuvant and additive materials.

11. Medicament according to claim 10 with calcium-regulating action.

12. Use of hPTH modifications according to one of claims 1 - 8 for the production of medicaments for the regulation of the calcium level in the body or for the incorporation of calcium into the bones.

## Revendications (Revendications pour l'(les) Etat(s) contractant(s) suivant(s): BE, DE, DK, ES, FR, GB, GR, NL, SE)

1. hPTH modifiées choisies dans le groupe constitué par les PTH(2-35), PTH(2-36), PTH(3-35) et PTH(3-36).

2. hPTH modifiées selon la revendication 1, caractérisées par le fait que les PTH modifiées sont la PTH(2-36) ou la PTH(2-36).

3. hPTH modifiées selon la revendication 1, caractérisées par le fait que les PTH modifiées sont la PTH(3-35) ou la PTH(3-36).

4. hPTH modifiées selon l'une quelconque des revendications 1-3, caractérisées par le fait que les groupes carboxy de l'extrêmité C-terminale sont amidés par un groupe Z et que Z représente un groupe de formule -CONR¹R², où R¹ et R² sont identiques ou différents et représentent un atome d'hydrogène ou un groupe alkyle en C₁-C₆.

5. hPTH modifiées selon la revendication 4, caractérisées par le fait que les PTH rmodifiées sont les PTH(2-35)-Z, PTH(2-36)-Z, PTH(3-35)-Z ou PTH(3-36)-Z.

6. Procédé de préparation de hPTH modifiées selon l'une quelconque des revendications 1-5, caractérisé par le fait que l'on prépare les fragments, selon la synthèse en phase solide ou liquide, à partir d'acides aminés partiellement bloqués, contenus dans les fragments, que l'on lie entre eux par couplage dans l'ordre correspondant aux séquences des acides aminés dans les fragments.

7. Médicament contenant des hPTH modifiées selon l'une quelconque des revendications 1-5, en tant que principes actifs, en plus d'adjuvants et additifs usuels.

8. Médicament selon la revendication 7, ayant une action régulatrice du calcium.

9. Utilisation des hPTH modifiées selon l'une quelconque des revendications 1-5, pour la préparation de médicaments destinés à la régulation du taux de calcium corporel ou pour l'incorporation de calcium dans les os.

## Revendications (Revendications pour l'(les) Etat(s) contractant(s) suivant(s): AT, CH, IE, IT, LI, LU, PT)

1. hPTH modifiées choisies dans le groupe constitué par les PTH(1-35), PTH(1-36), PTH(2-35), PTH(2-36), PTH(3-35) et PTH(3-36).

2. hPTH modifiées selon la revendication 1, caractérisées par le fait que la PTH modifiée est la PTH(1-35).

3. hPTH modifiées selon la revendication 1, caractérisées par le fait que la PTH modifiée est la PTH (1-36).

4. hPTH modifiées selon la revendication 1, caractérisées par le fait que les PTH modifiées sont la PTH (2-35) ou la PTH(2-36).

5. hPTH modifiées selon la revendication 1, caractérisées par le fait que les PTH modifiées sont la PTH (3-35) ou la PTH(3-36).

6. hPTH modifiées selon l'une quelconque des revendications 1-5, caractérisées par le fait que les groupes carboxy de l'extrêmité C-terminale sont amidés par un groupe Z et que Z représente un groupe de formule -CONR¹R², où R¹ et R² sont identiques ou différents et représentent un atome d'hydrogène ou un groupe alkyle en C₁-C₆.

7. hPTH modifiées selon la revendication 6, caractérisées par le fait que les PTH modifiées sont la PTH (1-35)-Z ou la PTH(1-36)-Z.

8. hPTH modifiées selon la revendication 6, caractérisées par le fait que les PTH modifiées sont les PTH (2-35)-Z, PTH(2-36)-Z, PTH(3-35)-Z ou PTH(3-36)-Z.

9. Procédé de préparation de hPTH modifiées selon l'une quelconque des revendications 1-8, caractérisé par le fait que l'on prépare les fragments, selon la synthèse en phase solide ou liquide, à partir des acides aminés bloqués partiellement, contenus dans les fragments, que l'on lie entre eux par couplage, dans l'ordre correspondant aux séquences des acides aminés dans les fragments.

10. Médicament contenant des hPTH modifiées selon l'une quelconque des revendications 1-8, en tant que principes actifs, en plus d'adjuvants et additifs usuels.

11. Médicament selon la revendication 10, ayant une action régulatrice du calcium.

12. Utilisation de hPTH modifiées selon l'une quelconque des revendications 1-8, pour la préparation de médicaments destinés à la régulation du taux de calcium corporel ou pour l'incorporation de calcium dans les os.


### SEQUENZPROTOKOLL

(1) ALGEMEINE INFORMATION:
   (i) ANMELDER:
      (A) NAME: Boehringer Mannheim GmbH
      (B) STRASSE: Sandhofer Str. 116
      (C) ORT: Mannheim 31
      (E) LAND: Germany
      (F) POSTLEITZAHL: 6800
      (G) TELEPHON: 0621/759-2019
      (H) TELEFAX: 0621/759-4457
   (ii) ANMELDETITEL: Neue Parathormonfragmente, deren Herstellung und diese enthaltende Arzneimittel
   (iii) ANZAHL DER SEQUENZEN: 4
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
   (vi) FRÜHERE ANMELDEDATEN:
      (A) ANMELDENUMMER: DE P 42 03 040.4
      (B) ANMELDEDATUM: 16-FEB-1992
(2) INFORMATION ZU SEQ ID NO: 1:
   (i) SEQUENZ CHARAKTERISTIKA:
      (A) LÄNGE: 33 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 1:
   Ser Glu Ile Gln Leu Met His Asn Leu Gly Lys His Leu Asn Ser Met
   1               5                   10                  15
   Glu Arg Val Glu Trp Leu Arg Lys Lys Leu Gln Asp Val His Asn Phe
               20                  25                  30
   Val
(2) INFORMATION ZU SEQ ID NO: 2:
   (i) SEQUENZ CHARAKTERISTIKA:
      (A) LÄNGE: 34 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 2:
   Ser Glu Ile Gln Leu Met His Asn Leu Gly Lys His Leu Asn Ser Met
   1 5 10 15
   Glu Arg Val Glu Trp Leu Arg Lys Lys Leu Gln Asp Val His Asn Phe
   20 25 30
   Val Ala
(2) INFORMATION ZU SEQ ID NO: 3:
   (i) SEQUENZ CHARAKTERISTIKA:
      (A) LÄNGE: 35 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 3:
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val
   35
(2) INFORMATION ZU SEQ ID NO: 4:
   (i) SEQUENZ CHARAKTERISTIKA:
      (A) LÄNGE: 36 Aminosäuren
      (B) ART: Aminosäure
      (C) STRANGFORM: Einzel
      (D) TOPOLOGIE: linear
   (ii) ART DES MOLEKÜLS: Peptid
   (xi) SEQUENZBESCHREIBUNG: SEQ ID NO: 4:
   Ser Val Ser Glu Ile Gln Leu Met His Asn Leu Gly Lys His Leu Asn
   1               5                   10                      15
   Ser Met Glu Arg Val Glu Trp Leu Arg Lys Lys Leu Gln Asp Val His
               20                  25                  30
   Asn Phe Val Ala
           35